(19) **Europäisches Patentamt European Patent Office Office européen des brevets**

(11) **EP 4 393 512 B1**

(12) **EUROPEAN PATENT SPECIFICATION**

(45) Date of publication and mention of the grant of the patent:
**01.10.2025 Bulletin 2025/40**

(51) International Patent Classification (IPC):
*A61K 9/20* (2006.01)    *A61K 31/155* (2006.01)
*A61P 3/10* (2006.01)

(21) Application number: **22861344.4**

(52) Cooperative Patent Classification (CPC):
**A61K 9/2027; A61K 9/2054; A61K 31/155; A61P 3/10**

(22) Date of filing: **23.08.2022**

(86) International application number:
**PCT/JP2022/031674**

(87) International publication number:
**WO 2023/027056 (02.03.2023 Gazette 2023/09)**

(54) **COMPOSITION FOR PHARMACEUTICAL TABLET, PHARMACEUTICAL TABLET USING THE SAME, AND MANUFACTURING METHOD THEREOF**

ZUSAMMENSETZUNG FÜR EINE PHARMAZEUTISCHE TABLETTE, DAMIT HERGESTELLTE PHARMAZEUTISCHE TABLETTE UND HERSTELLUNGSVERFAHREN DAFÜR

COMPOSITION POUR COMPRIMÉ PHARMACEUTIQUE, COMPRIMÉ PHARMACEUTIQUE L'UTILISANT, ET PROCÉDÉ DE SA PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2021 JP 2021136767**

(43) Date of publication of application:
**03.07.2024 Bulletin 2024/27**

(73) Proprietor: **Mitsubishi Chemical Corporation**
**Chiyoda-ku**
**Tokyo 100-8251 (JP)**

(72) Inventors:
• **KOBAYASHI, Ayaka**
**Tokyo 100-8251 (JP)**
• **YOSHIMURA, Nobuyoshi**
**Tokyo 100-8251 (JP)**

(74) Representative: **Vossius & Partner**
**Patentanwälte Rechtsanwälte mbB**
**Siebertstrasse 3**
**81675 München (DE)**

(56) References cited:
**EP-A1- 3 173 101        WO-A1-2016/013675**
**JP-A- 2020 114 816      US-A1- 2017 216 213**

• **ETSURO AWATA , YOSHIHIKO IKEGAMI: "Significance of Granulation in Pharmaceuticals", JOURNAL OF THE JAPAN SOCIETY OF POWDER AND POWDER METALLURGY, FUNTAI FUNMATSU YAKIN KYOKAI, JP, vol. 11, no. 6, 25 December 1964 (1964-12-25), JP**
**, pages 307 - 315, XP009543811, ISSN: 0532-8799, DOI: 10.2497/jjspm.11.307**
• **ANONYMOUS: "Effect of Particle Size Distribution on Powder Packability (Vacancy Ratio)", 1 January 2015 (2015-01-01), XP093039582, Retrieved from the Internet <URL:https://web.archive.org/web/20150806200040/https://www.eng.u-hyogo.ac.jp/group/group42/hakaru/eikyo.html> [retrieved on 20230417]**

**Description**

**TECHNICAL FIELD**

The present invention relates to a composition for a pharmaceutical tablet containing a polyvinyl alcohol-based resin and crystalline cellulose, a pharmaceutical tablet using the same, and a method for producing the pharmaceutical tablet.

**BACKGROUND**

Oral agents such as tablet, capsule, granule, and powder are now widely used as a dosage form of pharmaceutical. Of these, the tablet is generally manufactured by tableting, which comprises filling into die either mixed powder of an active ingredient (pharmaceutical ingredient) with various additive materials, or granules made from the mixed powder, and pressing with a punch to form into a desired size and shape. Thus manufactured pharmaceutical tablet may also be coated, if desired.

By the way, a sustained-release solid dosage form such as tablet can be controlled in release rate of a pharmaceutical ingredient so as to extend the release time of the pharmaceutical ingredient. Such a tablet may reduce frequency of administration and thereby improving medication compliance, moreover, may lessen fluctuations in blood concentration and thereby lowering a side effect. Therefore, a sustained-release solid dosage form has been studying and developing in recent years.

A tablet may contain additives such as excipient, binder, disintegrant, lubricant, and the like. The excipient does not have a physiological activity and may be added in an amount sufficient for assuring handleability during manufacturing processes. The binder may be added for binding particles of ingredient powder and may control the mechanical strength of a resultant tablet. The disintegrant, which can disintegrate the tablet by swelling due to absorption of water in the body, may be added so as to enhance the release of the pharmaceutical ingredient from the tablet. The lubricant may be added so as to run the ingredient powder smoother and facilitate a compressing operation for tableting.

A polyvinyl alcohol-based resin or PVA-based resin is water-soluble and biodegradable, and moreover can act as a binder for a wide variety of ingredients. Based on this property, PVA-based resin is commonly used as an additive for a pharmaceutical tablet, particularly as a binder, coating agent for film coated tablet etc. Among the above-mentioned additives, a binder and a disintegrant are known to affect not only the strength of a resulting tablet but also release rate of the pharmaceutical ingredient. Therefore, an application of polyvinyl alcohol-based resin to the preparation of a sustained-release solid dosage form has been investigated.

For example, WO 2016/013675 (Patent Document 1) suggested a sustained-release tablet. The sustained-release tablet is imparted with a sustained-release property by containing a PVA fine particle having a gauche structure in a specific amount or more in the surface of the particle. The PVA fine particle was used as a binder to enhance adhesion between ingredients in the tablet. In the Patent Document 1, a tablet was prepared by adding a PVA fine particle (ground powder having 96 $\mu$m as a median or 50% diameter in particle size distribution) having a saponification degree of 88% and an average polymerization degree of 2400, to a wet granulate blend of a pharmaceutical ingredient and crystalline cellulose, and subsequently mixing with crystalline cellulose, aerosil, and magnesium stearate to undergo compression molding. Thus produced tablet exhibited sustained-release as compared to a tablet containing a PVA fine particle (ground powder having 100 $\mu$m as a median or 50% diameter in particle size distribution) having a lower proportion of gauche structure (Fig. 5). In the examples, metformin hydrochloride was used as a pharmaceutical ingredient.

By the way, a solid dosage form of medicament such as pharmaceutical tablet should comply with an established formulation standard for physical properties including hardness and friability. A pharmaceutical excipient is required to meet the ranges identified by the formulation standard. However, the tablet disclosed in Patent Document 1 did not have a sufficient hardness satisfying the requirements. If a sustained-release tablet is chewed or crushed in the oral cavity, the pharmaceutical ingredient is released rapidly at an initial stage. As a result, the sustained-release tablet fails to fulfill its role, to make the matters worse, a side effect might be raised. For these reasons, a sustained-release tablet needs to increase its hardness for attaining a practical use.

On the other hand, JP 2018-530537 A (Patent document 2) describes that a high hardness tablet can be obtained by compressing a combination (dry co-mixture) of PVA (an average particle size of less than 100 $\mu$m) and microcrystalline cellulose which are to be contained as a matrix. The tablet disclosed in Patent Document 2 is a sustained-release tablet capable of releasing the pharmaceutical ingredient over a long time period independently of the composition of the release medium.

In a formulation containing a pharmaceutical ingredient from BCS class I, a mixture of said PVA and said microcrystalline cellulose was employed in the ratio from 1:0.5 to 1:2. Although the pharmaceutical ingredient from BCS class I is an active ingredient having high solubility and high permeability, the formulation exhibited a delayed release independently of the composition of the release medium.

## PRIOR ART

### PATENT DOCUMENT

[Patent Document 1] WO 2016/013675
[Patent Document 2] JP 2018-530537 A

## SUMMARY OF THE INVENTION

### TECHNICAL PROBLEM TO BE SOLVED BY THE INVENTION

In the Patent Document 2, a pharmaceutical formulation prepared by compressing a mixture of PVA and microcrystalline cellulose at a compression force selected from 10 kN, 20 kN, and 30 kN, was measured with respect to a release rate with use of some types of medium. As the PVA, PVAs selected from grade 26 - 88 (which has a saponification degree of 88% and a viscosity of 26 mPa • s measured in 4% aqueous solution) and grade 40 - 88 (which has a saponification degree of 88% and a viscosity of 40 mPa • s measured in 4% aqueous solution) were used wherein the grade is in accordance with the requirements of Ph.Eur. (European Pharmacopeia), USP (United States Pharmacopeia), or Japan Pharmaceutical Excipients Standard (JPE). The PVA was a ground PVA powder having an average particle-size fraction (Dv50) of 85 $\mu$m to 88 $\mu$m. The microcrystalline cellulose had an average particle-size fraction (Dv50) of 100 $\mu$m to 140 $\mu$m.

The Patent Document 2 discloses that the tablet hardness is dependent on compression force (Fig. 1), and a tablet produced with a compression force of 20 kN or more with the powder having a particle size falling in said range could achieve the hardness of 200 N or more.

On the other hand, the graphs (Fig. 2a, Fig. 2b, and Fig. 2c) show that as the compression force increases to 10 kN (Fig. 2a), 20 kN (Fig. 2b), and 30 kN (Fig. 2c), the release rate decreases after 10 hours of its administration. This is further supported by the data of phosphate buffer in Tables 2a, 2b, and 2c, where the average values after 10 hours decreased by 89%, 88%, and 85%, and the maximum values after 10 hours decreased by 91%, 90%, and 87% respectively. Since the release amount of the pharmaceutical ingredient did not reach 100% even after 12 hours, the release of the pharmaceutical ingredient seems to be hindered.

The present invention has been made in view of such circumstances. The objective is to provide a composition for sustained-release tablet which is too hard to chew, and thereby suppressing release of the pharmaceutical ingredient for a few hours after administration, but can release 90% or more of the pharmaceutical ingredient within 10 hours after administration. The objective is also to provide a sustained-release tablet using the same and a method for producing the same.

### MEANS FOR SOLVING THE PROBLEM

The composition for a pharmaceutical tablet of the disclosure comprises a polyvinyl alcohol-based resin and crystalline cellulose, wherein the polyvinyl alcohol-based resin has an average particle size of 100 $\mu$m or less and a viscosity of more than 44 mPa • s measured in 4% aqueous solution.

In a preferable embodiment, the crystalline cellulose has an average particle size of 250 $\mu$m or less, and the average particle size is either up to half or at least twice the size of an average particle size of the polyvinyl alcohol-based resin.

The weight ratio of the polyvinyl alcohol-based resin to the crystalline cellulose (polyvinyl alcohol-based resin/crystalline cellulose) is preferably from 100/200 to 100/1. In a more preferred embodiment, the weight ratio of the polyvinyl alcohol-based resin to the crystalline cellulose (polyvinyl alcohol-based resin/crystalline cellulose) is greater than 2/1 but 50/1 or less.

The polyvinyl alcohol-based resin and the crystalline cellulose are preferably blended so that the tapped density is in the range between 0.50 and 0.68 g/mL.

In one embodiment of the composition for a pharmaceutical tablet of the disclosure, the composition comprises a polyvinyl alcohol having a viscosity greater than 44 mPa • s measured in 4% aqueous solution and having an average particle size of 100 $\mu$m or less, and a crystalline cellulose having an average particle size of 250 $\mu$m or less.

The average particle size of the crystalline cellulose is either not more than 0.5 times or not less than twice the size of the average particle size of the polyvinyl alcohol-based resin, and the blending ratio in weight of the polyvinyl alcohol-based resin and the crystalline cellulose (polyvinyl alcohol-based resin/crystalline cellulose) is greater than 2/1 but 50/1 or less.

In the composition for pharmaceutical tablet of the disclosure, the saponification degree of the polyvinyl alcohol-based resin is preferably 70 mol % or more but 100 mol % or less.

The present invention also includes a pharmaceutical tablet comprising the above-mentioned composition for pharmaceutical tablet and a pharmaceutical ingredient classified into either class I or III in accordance with BCS classification. Said pharmaceutical tablet is preferably a sustained-release tablet.

Another aspect of the invention includes a method for producing a pharmaceutical tablet. The method for producing a pharmaceutical tablet of the invention comprises tableting a mixture which contains a pharmaceutical ingredient; a crystalline cellulose; and a polyvinyl alcohol-based resin having an average particle size of 100 $\mu$m or less and a viscosity greater than 44 mPa · s measured in 4% aqueous solution.

The pharmaceutical ingredient is preferably a granule granulated using a binder.

In this disclosure, the average saponification degree is referred as a saponification degree measured in accordance with JIS K 6726 (1994).

The "viscosity measured in 4% aqueous solution at 20°C" referred in the specification can be measured by the following method. 500 g of water and 25 g of PVA-based resin are mixed, stirred at a temperature of 60 to 100°C for 60 minutes to dissolve completely, and followed by adding water to prepare an aqueous solution having a concentration of 4% by weight. This 4% aqueous solution is kept in a water bath having a constant temperature of 20°C for 30 minutes or more. After confirming that the temperature of the aqueous solution reaches 20°C, the viscosity of the 4% aqueous solution is measured by the falling ball viscometer method according to JIS K6726.

The average polymerization degree in this specification is referred as an average polymerization degree (number average polymerization degree) measured in accordance with JIS K 6726 (1994). In a tablet containing two or more types of polyvinyl alcohol-based resins differing in average polymerization degree, the overall average polymerization degree corresponds to the sum of the individual number average polymerization degrees, taking into consideration their content rates.

The average particle size in this specification is an average value of particle sizes measured by a laser diffraction method.

## EFFECT OF THE INVENTION

A composition for a pharmaceutical tablet of the present invention can provide a tablet with such an increased hardness that the tablet has a high chewing difficulty. In addition, the hydrogel formed from the PVA-based resin can suppress the release of the pharmaceutical ingredient. Therefore, even if a readily soluble pharmaceutical ingredient is employed, the composition may give an excellent sustained-release property.

## EMBODIMENT FOR CARRYING OUT THE INVENTION

[Composition for a pharmaceutical tablet]

The composition for a pharmaceutical tablet of the invention comprises (A) a polyvinyl alcohol-based resin used as a matrix material and (B) a crystalline cellulose, wherein the polyvinyl alcohol-based resin (A) has a specific range of particle size and a specific range of viscosity measured in 4% aqueous solution.

Each component will be described in detail below.

(A) Polyvinyl alcohol-based resin used as a matrix material

A polyvinyl alcohol (PVA)-based resin used in the composition for solid dosage form of the disclosure is a resin obtained by saponification of a polyvinyl ester-based resin which is a polymer of a vinyl ester-based monomer. The PVA-based resin is a resin mainly composed of a vinyl alcohol structural unit because the vinyl alcohol structural unit is contained in an amount corresponding to the saponification degree. The PVA-based resin optionally contains a vinyl ester structural unit as an unsaponified portion. The polyvinyl alcohol (PVA)-based resin used in the composition for solid dosage form is not limited to an unmodified PVA-based resin consisting of a vinyl alcohol unit and a vinyl ester unit. A modified polyvinyl alcohol-based resin or a post-modified PVA-based resin may be used. A modified polyvinyl alcohol-based resin is a saponified copolymer of vinyl ester-based monomer and copolymerizable monomer with the vinyl ester-based monomer. In the case of the modified PVA-based resin, the modification degree, which is correspondent to a content of structural units other than a vinyl alcohol unit and a vinyl ester structural unit, is 20 mol % or less, preferably 1 mol % or more and 10 mol % or less, more preferably 1 mol % or more and 5 mol % or less.

Examples of the vinyl ester monomer include vinyl formate, vinyl acetate, vinyl propionate, vinyl valerate, vinyl butyrate, vinyl isobutyrate, vinyl pivalate, vinyl caprate, vinyl laurate, vinyl stearate, vinyl benzoate, vinyl versatate and the like. Of these vinyl esters, vinyl acetate is preferably used for a practical reason.

Examples of the copolymerizable monomer include olefins such as ethylene, propylene, isobutylene, α-octene, α-dodecene, and α-octadecene; hydroxy group-containing α-olefins such as 3-buten-1-ol, 4-penten-1-ol, 5-hexen-1-ol, 3,4-dihydroxy-1-butene and their derivatives such as acylated products; unsaturated acids such as acrylic acid, methacrylic acid, crotonic acid, maleic acid, maleic anhydride, itaconic acid, undecylenic acid, and salts, monoesters, or dialkyl esters thereof; nitriles such as acrylonitrile and meta-acrylonitrile; amides such as diacetoneacrylamide acrylamide and methacrylamide; olefin sulfonic acids such as ethylene sulfonic acid, allyl sulfonic acid, methallyl sulfonic acid or salts thereof; alkyl vinyl ethers; vinyl compounds such as dimethylallyl vinyl ketone, N-vinylpyrrolidone, vinyl chloride, vinylethylene carbonate, 2,2-dialkyl-4-vinyl-1,3-dioxolane, and glycerin monoallyl ether; substituted vinyl acetates such as isopropenyl acetate and 1-methoxyvinyl acetate, vinylidene chloride, 1,4-diacetoxy-2-butene, 1,4-dihydroxy-2-butene, vinylene carbonate and the like. Such copolymerizable monomers may be used alone or in combination of two or more of them.

As the modified PVA-based resin obtained by copolymerization with an unsaturated acid, or salt, monoester, or dialkyl ester thereof, for example, polyvinyl alcohol/acrylic acid/methyl methacrylate copolymer is preferable.

A modified PVA-based resin having a primary hydroxyl group in the side chain is also preferably used. Such a modified PVA-based resin can be obtained by using a copolymerizable monomer such as 3,4-diacetoxy-1-butene, vinyl ethylene carbonate, or glycerin monoallyl ether; or a copolymerizable monomer having a hydroxymethyl group in a side chain such as hydroxymethylvinylidene diacetate (e.g., 1,3-diacetoxy-2-methylenepropane, 1,3-dipropionyloxy-2-methylenepropane, 1,3-dibutyronyloxy-2-methylenepropane). A modified PVA-based resin having 1,2-diol bond in the side chain can be obtained by a method, for example, (a) a method of saponifying a copolymer of a vinyl ester-based monomer and 3,4-diacetoxy-1-butene, (b) a method of saponifying and decarboxylating a copolymer of a vinyl ester-based monomer and vinyl ethylene carbonate, (c) a method of saponifying and deketalizing a copolymer of a vinyl ester-based monomer and 2,2-dialkyl-4-vinyl-1,3-dioxolane, (d) a method of saponifying a copolymer of a vinyl ester-based monomer and glycerin monoallyl ether, and so on.

The modified PVA-based resin may be a post-modified PVA-based resin obtained by post-modifying an unmodified PVA. A post-modification method includes acetoacetic esterification, acetalization, urethanization, etherification, phosphate esterification, oxyalkylenation, and the like.

The vinyl ester-based monomer (optionally including a copolymerizable monomer) can be polymerized by a conventionally known method (bulk polymerization, solution polymerization, suspension polymerization, dispersion polymerization, emulsion polymerization, etc.). As a polymerization catalyst and a solvent used in the polymerization, a conventionally known one may also be used. In general, an alcohol, preferably a lower alcohol having 1 to 3 carbon atoms, is used.

Saponification of the resulting vinyl ester-based polymer can also be carried out by a conventionally known method. Industrially, the polymer can be dissolved in an alcohol or water/alcohol solvent using an alkali catalyst or an acid catalyst.

A common alkali catalyst includes a hydroxide or an alcoholate of an alkali metal such as potassium hydroxide, sodium hydroxide, sodium methylate, sodium ethylate, potassium methylate and lithium methylate.

Saponification is usually carried out by transesterification using an alkali catalyst, preferably in the presence of an anhydrous alcoholic solvent, from the viewpoint of the reaction rate and the reduction of impurities such as fatty acid salts.

The reaction temperature for the saponification is usually selected from the range of 20 to 60°C. Unduly low reaction temperature may cause to lower the reaction rate and reaction efficiency. Unduly high reaction temperature may be concerned with that the reaction temperature is higher than the boiling point of the reaction solvent. This may impair safety in a manufacturing process. In the case that saponification is performed under high pressure using a high pressure-proof column type continuous saponification tower, a temperature as high as the temperature ranging from 80 and 150°C can be selected. In this case, a high saponification degree can be achieved even in a shortened time and with a reduced amount of catalyst.

Preferably, the PVA-based resin obtained by saponification is cleansed with a liquid. Examples of the cleansing liquid include alcohols such as methanol, ethanol, isopropyl alcohol, and butanol. Of these, methanol is preferable for striking the balance between effective cleansing and cost-efficient drying.

The cleansing process is usually conducted at a temperature of 10 to 80°C, preferably 20 to 70°C, for 5 minutes to 12 hours.

The PVA-based resin cleansed with a lower alcohol such as methanol is dried with hot air or the like in a continuous or batch manner to provide PVA-based resin powder. The drying process is usually conducted at a temperature of 50 to 150°C, preferably 60 to 130°C, particularly preferably 70 to 110°C, for 1 to 48 hours, preferably 2 to 36 hours.

The PVA-based resin after the drying process commonly contains the solvent in the range between 0% by weight or more and 10% by weight or less, preferably 0.1% by weight or more and 5% by weight or less, and more preferably 0.1% by weight or more and 1% by weight or less.

The average saponification degree of the PVA-based resin used in the disclosure is in the range of 78 to 96 mol%, and preferably 85 to 89 mol%. This range meets the saponification degree of a partially saponified polyvinyl alcohol specified in the international standard including Japan Pharmaceutical Excipients Standard (JPE), the European Pharmacopeia (EP) and the United States Pharmacopeia (USP).

PVA-based resin having an average saponification degree within the said range may exhibit high water solubility, so the PVA-based resin contained in the orally administrated tablet may form hydrogen bonds with hydroxyl groups of crystalline cellulose to create a gel which contributes to sustained-release of a pharmaceutical ingredient from the tablet. On the other hand, PVA-based resin having unduly low saponification degree has a tendency to form an insufficient gel due to the reduction of the hydroxyl groups, resulting in unsatisfactory sustained-release. Poor gel-forming ability may lower the resin in strength, which may be associated with a decrease in tablet hardness.

The number average polymerization degree of the PVA-based resin (measured according to JIS K 6726) is usually 2000 or more, preferably from 2200 or more to 4000 or less, more preferably from 2400 or more to 3500 or less, and particularly preferably from 2450 or more to 3000 or less. A PVA-based resin having unduly low number average polymerization degree tends to form a gel with poor density (or insufficient network density), which may not ensure a suppression of the initial release of the pharmaceutical ingredient.

The viscosity of the aqueous solution having a concentration of 4% by weight of the PVA-based resin used as the matrix is 44 mPa•s or more, preferably 45 mPa•s or more but 70 mPa•s or less, more preferably in the range of 46.5 mPa•s or more to 60 mPa•s or less, at 20°C. The aqueous solution (4 % by weight) having an unduly high viscosity is usually derived from a PVA-based resin having unduly high average polymerization degree. Therefore, a PVA-based resin having such unduly high viscosity measured in 4% aqueous solution has a tendency to lower compressibility. The aqueous solution (4 % by weight) having an unduly low viscosity is derived from PVA-based resin having unduly low average polymerization degree, and therefore the PVA-based resin might inhibit an increase in tablet hardness. In addition, as the PVA-based resin having unduly low average polymerization degree exhibits a relatively high water solubility, the initial release of the pharmaceutical ingredient tends to be enhanced, which means to impair a sustained release property of the tablet.

The PVA-based resin may be in the form of powder, granule or the like, preferably powder or granule. The average particle size of the PVA-based resin which is measured by laser diffraction method is 100 $\mu$m or less, preferably in the range from 30 $\mu$m or more to less than 100 $\mu$m, more preferably from 35 $\mu$m or more to 85 $\mu$m or less, still more preferably from 40 $\mu$m or more to 75 $\mu$m or less, most preferably from 40 $\mu$m or more to 60 $\mu$m or less.

A PVA-based resin having an unduly large average particle size might not have a necessary high bulk density, as a result, a tablet having a desired high hardness might not be produced. On the other hand, the PVA-based resin having an unduly low average particle size tends to be low productivity of the griding process of the PVA particles and to behave a particle with insufficient fluidity in the production of the tablet.

The PVA-based resin powder having an average particle size within the said range may be produced by synthesis or ground particles of the synthesized PVA-based resin. The PVA-resin powder produced by grinding process usually has a tapped density of 0.60 to 0.75 g/mL, preferably 0.61 to 0.68 g/mL.

An amount of the PVA-based resin contained in the pharmaceutical tablet is usually in the range from 5% by weight or more to 90% by weight or less, preferably 10% by weight or more to 80% by weight or less, and particularly preferably 20% by weight or more to 70% by weight or less, based on the weight of the tablet. If the amount is too low, a resulting tablet may not achieve an intended sustained-release property, and if the amount is too high, a resulting tablet would not have a sufficient hardness.

(B) Crystalline cellulose

Crystalline cellulose is contained as an excipient. Crystalline cellulose is excellent in compressibility, and therefore allows to increase the compression ratio in a production of the tablet. As a result, a hardness of the resulting tablet may be increased. Containing such crystalline cellulose may increase tablet hardness and chewability difficulty.

The average particle size of the crystalline cellulose used in the disclosure is usually 250 $\mu$m or less, but not limited thereto. Crystalline cellulose having unduly small average particle size may provide powder having insufficient fluidity. Crystalline cellulose having unduly large average particle size may be lowered in its compression moldability.

In a preferable embodiment, the average particle size (Dc) of the crystalline cellulose is different from the average particle size (Dp) of the matrix PVA-based resin. In a particularly preferable embodiment, the ratio of Dc and Dp, Dc/Dp, is 1.5 or more, 2 or more, but 5 or less, 4 or less. Alternatively, the ratio (Dc/Dp) is less than 0.7, more preferably 0.5 or less. Adjusting the relationship between their average particle sizes to the specified range can increase the hardness of the product produced by compression molding as described later, although the hardness depends on the content ratio of the PVA-based resin and the crystalline cellulose.

A preferable average particle size of the crystalline cellulose used in the disclosure falls within a specified range. In addition, a crystalline cellulose having the specified average particle size satisfies a tapped density of 0.50 to 0.68 g/mL, more preferably 0.52 to 0.65 g/mL, still more preferably 0.55 to 0.63 g/mL, as a combination with the matrix PVA-based resin (in a mixture state). The tapped density of the combination (mixture state) of the matrix PVA-based resin and the crystalline cellulose may usually be lower than the tapped density of the matrix PVA-based resin alone. On the other hand, a combination having different average particle sizes may make a broad distribution of the particle size of the mixture, and thereby allowing the mixture to reduce the porosity of the product produced by compression. This may achieve to produce a tablet having an increased hardness.

The content of the crystalline cellulose is not particularly limited, however, for ensuring a desired sustained-release property, a higher content of the PVA-based resin for the matrix than the content of the crystalline cellulose is preferable as compared with the case that these contents are equal. Specifically, the content ratio in weight of PVA-based resin and crystalline cellulose, PVA-based resin/crystalline cellulose, is usually in the range from 100/200 or more to 100/1 or less, for example, preferably more than 2/1, more preferably 3/1 or more, still more preferably 5/1 or more, but preferably 50/1 or less, more preferably 15/1 or less, still more preferably 10/1 or less.

By setting such a content ratio, the porosity in a combination or mixture of the two differing in average particle size can be decreased, and the tablet hardness can be increased. This may ensure a desired sustained-release property.

The composition containing the PVA-based resin and crystalline cellulose as the matrix material as described above can provide a hard tablet having chewing difficulty in the oral cavity despite the high content of the PVA-based resin. In addition, the release of the pharmaceutical ingredient in the early stage of administration is suppressed (such that a release rate after 1 hour from the administration is 55% or less, preferably 50% or less, and an increased amount of the release rate between 1 hour to 3 hours after administration is at most 35%, preferably 30% or less), but 90% or more of the pharmaceutical ingredient can be released 10 hours after administration.

By containing a relatively large amount of a PVA-based resin having a high number average polymerization degree or high viscosity measured in 4% aqueous solution as the matrix material, and at the same time employing a crystalline cellulose having an average particle size capable of increasing the density in a combination with the PVA-based resin, a tablet having a desired hardness can be produced and the tablet can form a dense gel sufficient to suppress the initial release of the pharmaceutical ingredient from the tablet even if the pharmaceutical ingredient has water-solubility such that it is classified into class I or class III in the BCS classification.

(C) Other materials

The composition for a solid dosage form of the disclosure may contain a material other than the said ingredients within a range that does not impair the effects of the disclosure, for example, 20% by weight or less, preferably 10% by weight or less, based on the total weight of the composition of the tablet. Examples of other materials include excipient, binder, disintegrant, pH adjuster, fluidizing agent, surfactant, coloring agent, sweetener and coating agent.

(C-1) Binder

A binder may be added to promote binding between particles in the process of dry or wet granulating, and tableting by direct compression or wet process. Such binder is appropriately used to control the disintegration of solid dosage form such as a tablet.

The binder contains in an amount of usually 0.1 to 10 parts by weight, preferably 0.2 to 5 parts by weight, and more preferably 0.5 to 3 parts by weight, per 100 parts by weight based on the total weight of the composition of the tablet, depending on the type of compound used.

The polyvinyl alcohol-based resin that can be used as a binder may be an unmodified PVA-based resin, or a modified PVA-based resin with a copolymerizable monomer as already mentioned for the polyvinyl alcohol-based resin used as a matrix material.

A polyvinyl alcohol-based resin used as a binder is preferably used in the form of aqueous solution.

The polyvinyl alcohol-based resin used as the binder may be distinguished from the polyvinyl alcohol-based resin contained as the matrix material in viscosity of 4% aqueous solution. The viscosity at 20°C of aqueous solution (4% by weight) of the polyvinyl alcohol resin as the binder is in the range from preferably 2 mPa • s or more to 30 mPa • s or less, more preferably 2 mPa • s or more to 20 mPa • s or less, and particularly preferably 2 mPa • s or more to 10 mPa • s or less. In general, a PVA-based resin used as a binder has a lower polymerization degree than the PVA-based resin used as a matrix material.

Regarding the saponification degree of the polyvinyl alcohol-based resin used as a binder, a preferable range is from 70 mol% or more to 99 mol% or less, a more preferable range is from 80 mol% or more to 90 mol% or less. These ranges are similar to that of the PVA-based resin for a matrix material.

When employing said polyvinyl alcohol-based resin for binder, a resulting tablet contains two or more types of polyvinyl alcohol-based resins differing in average polymerization degree. In the case that a tablet contains two or more types of polyvinyl alcohol-based resins differing in average polymerization degree, the overall average polymerization degree of the polyvinyl alcohol-based resins contained in the composition for solid dosage form is employed. The overall average polymerization degree corresponds to the sum of the individual average polymerization degrees calculated based on their content rates.

For example, in the case of containing two types of PVA-based resins, i.e. a matrix PVA-based resin and a binder PVA-based resin, the average saponification degree and average polymerization degree may be calculated respectively by the following formulas, wherein SDm and Pm are referred as a saponification degree and polymerization degree of matrix PVA-based resin respectively, SDb and Pb are referred as a saponification degree and polymerization degree of a binder PVA-based resin respectively, and M and B are the respective content rates of matrix PVA-based resin and binder PVA-based resin to the total amount of PVA-based resins. Note that M+B=1.

$$\text{Average saponification degree} = SDm \times M + SDb \times B$$

$$\text{Average polymerization degree} = Pm \times M + Pb \times B$$

The content of the PVA-based resin used for the binder (B) is typically at most about 5% by weight (usually 3% by weight or less, preferably 2.5% by weight or less) based on the total weight of PVA-based resins. Therefore, even if the binder PVA-based resin having an average polymerization degree less than 800 is contained, the measurement value of the average polymerization degree of the PVA-based resin contained in the composition for solid dosage form may be 2000 or more.

(C-2) Other binders

As a binder other than the above-mentioned PVA-based resin binder, dextrin, gum arabic, gelatin, hydroxypropyl starch, methyl cellulose, hydroxypropyl cellulose, hypromellose, pullulan, starch paste, and the like may be used.

(C-3) Other additives

A lubricant may be added so as to improve fluidity, provide lubricity during compression in the die and during tablet ejection, and avoid sticking to the punch faces or die wall. Examples of the lubricant include stearic acid, magnesium stearate, calcium stearate, talc, anhydrous silicic acid, and the like.

Other excipients that can be used include sugar alcohols (such as mannitol, erythritol, xylitol, sorbitol and maltitol), sugars (such as glucose, fructose, lactose, sucrose, trehalose, maltose, and oligosaccharides), calcium phosphates, starches, sodium phosphates and gelatin. Among these, a sugar alcohol, in particular, mannitol is preferably used.

Furthermore, if necessary, disintegrants (e.g., carmellose calcium, carboxymethyl starch sodium, croscarmellose sodium, crospovidone, non-crystalline cellulose or derivatives thereof, and starch or derivatives thereof); pH adjusters (e.g., citric acid and its salts, phosphoric acid and its salts, carbonic acid and its salts, tartaric acid and its salts, fumaric acid and its salts, acetic acid and its salts, amino acids and its salts, succinic acid and its salts and lactic acid and its salt); flow agents (e.g., light anhydrous silicic acid, hydrated silicon dioxide, titanium oxide, stearic acid, corn gel, and heavy silicic anhydride); surfactants (e.g., phospholipids, glycerin fatty acid esters, polyoxyethylene fatty acid esters, sorbitan fatty acid esters, polyethylene glycol fatty acid esters, polyoxyethylene hydrogenated castor oil, polyoxyethylene alkyl ethers, sucrose fatty acid esters, sodium lauryl sulfate, polysorbates, sodium hydrogen phosphates and potassium hydrogen phosphates); coloring agents (e.g., iron sesquioxide, yellow iron sesquioxide, food yellow No. 5, food yellow No. 4, aluminum chelate, titanium oxide, and talc); and sweeteners (e.g., saccharin, aspartame, acesulfame potassium, thaumatin and sucralose) may be contained.

[Sustained-release tablet]

The sustained-release tablet of the present invention is a tablet obtained by adding a pharmaceutical ingredient to a composition for a pharmaceutical tablet and tableting.

By using an inventive composition for a pharmaceutical tablet, the release rate and release time of the active pharmaceutical ingredient from the tablet can satisfy a sustained-release property in accordance with the Japanese Pharmacopeia 18th edition, to reduce the frequency of administrations or mitigate a side effect.

[Active pharmaceutical ingredient (API)]

Examples of the active pharmaceutical ingredient applicable to the solid dosage form of the disclosure include antipyretic analgesic antiphlogistics, nutrient and tonic supplements, psychotropics, antidepressants, antianxiety drugs, hypnosedatives, anticonvulsants, CNS-acting drugs, brain metabolism improving agents, brain circulation improving agents, antiepileptic agents, sympathomimetic drugs, gastrointestinal drugs, acid suppressants, anti-ulcerogenic drugs, cough medicines, antiemetics, anapnoics, bronchodilators, allergic drugs, antihistamine agents, agents for dental administration or oral administration, cardiants, agents for cardiac arrhythmia, diuretics, hypertension drugs, vasoconstrictors, coronary vasodilators, peripheral vasodilators, blood coagulation inhibitors, hyperlipidemias agents, cholagogues, antibiotics, chemotherapeutic agents, diabetes drugs, osteoporosis drugs, antirheumatics, skeletal muscle relaxants, antispasmodics, hormonal agents, alkaloid drugs, sulfa drugs, arthrifuges, and antineoplastics.

The composition for a tablet of the disclosure can provide a tablet having a high hardness and impart a sustained-release property. Therefore, it is effective to combine with a pharmaceutical ingredient that requires sustained-release property, especially a pharmaceutical ingredient for oral administration that requires sustained-release property at an initial stage of administration due to its readily water-solubility, in particular, a readily water-soluble pharmaceutical ingredient which may raise a side effect in addition to reduction of sustained-release property when the tablet is crushed by chewing.

In this specification, a readily water-soluble pharmaceutical ingredient is a medicament classified as very soluble, freely soluble, or soluble in water in accordance with the Japanese Pharmacopeia 18th edition, or class I or III in the BCS (Biopharmaceutical Classification System).

The BCS is a system for classifying pharmaceutical ingredients based on their solubility and membrane permeability. A pharmaceutical ingredient of class I has high solubility and high membrane permeability, while a pharmaceutical ingredient of class III has high solubility but low membrane permeability.

Examples of readily water-soluble pharmaceutical ingredients, specifically, a pharmaceutical ingredient that can be completely dissolved or highly soluble at a pH of 1.2 to 6.8 at 37 $\pm$ 1°C, in at most 250 mL of an aqueous solvent, include metformin hydrochloride, sodium salicylate, aminopyrine, benzydamine hydrochloride, tiaramide hydrochloride, doxycycline hydrochloride hydrate, moxalactam sodium, bacampicillin hydrochloride, levamisole hydrochloride, dl-methylephedrine hydrochloride, noscapine, codeine phosphate hydrate, dihydrocodeine phosphate, cloperastine hydrochloride, dl-isoprenaline hydrochloride, L-ethylcysteine hydrochloride, L-methylcysteine hydrochloride, tolperisone hydrochloride, eperisone hydrochloride, tizanidine hydrochloride, roxatidine acetate hydrochloride, chlorpheniramine maleate, diphenhydramine hydrochloride, imipramine hydrochloride, clomipramine hydrochloride, clemizole hydrochloride, methoxyphenamine hydrochloride, etilefrine hydrochloride, alprenolol hydrochloride, bufetrol hydrochloride, oxprenolol hydrochloride, procainamide hydrochloride, disopyramide phosphate, mexiletine hydrochloride, diltiazem hydrochloride, isosorbide mononitrate, dilazep hydrochloride hydrate, hexamethonium bromide, clonidine hydrochloride, buformin hydrochloride, isoniazid, ethambutol hydrochloride, thiamazole, vitamin C, B1, or B2, loxoprofen sodium hydrate, oxybutynin hydrochloride and the like.

The content of the active pharmaceutical ingredient (API) as exemplified above is usually 0.5/100 or more but 1000/100 or less, preferably 20/100 or more but 500/100 or less, more preferably 30/100 or more but 300/100 or less, in terms of matrix PVA-based resin/API (weight ratio).

[Pharmaceutical tablet and manufacturing method thereof]

The method for producing a pharmaceutical tablet of the present invention comprises mixing the inventive composition for pharmaceutical tablet with a pharmaceutical ingredient, and tableting the mixture.

Regarding the mixture, the content ratio of crystalline cellulose and PVA-based resin, PVA-based resin/crystalline cellulose (weight ratio), is in the range between 100/1 and 100/200, preferably the range from more than 2/1, more preferably 3/1 or more, still more preferably 5/1 or more, but preferably up to 50/1 or less, more preferably 15/1 or less, still more preferably 10/1 or less.

Moreover, regarding the ratio of the average particle size between of the crystalline cellulose (Dc) and the matrix PVA-based resin (Dp), Dc/Dp, a preferable mixture satisfies the ratio of either the range from 1.5/1 to 5/1, or not more than 0.5.

In the tableting process, the mixture further containing various may be compressed into a tablet directly. Alternatively, the mixture may be granulated and then the obtained granules may be compressed into a tablet by punching.

In the case that a tablet is formed from granules, it is preferable to first produce a granule containing the binder and the pharmaceutical ingredient, mix the granules with powder of the other ingredients such as PVA-based resin for matrix and crystalline cellulose, and thereafter compress the mixture into tablet.

In a pharmaceutical tablet of the invention produced by compression, the pharmaceutical ingredient is homogeneously embedded in the matrix PVA-based resin. By use of a mixture comprising granules of the pharmaceutical ingredient granulated with a binder, and a matrix including a matrix PVA-based resin and crystalline cellulose, the pharmaceutical ingredient may be confined in the gel formed by the matrix PVA-based resin. Thus the tablet may ensure a sustained-release performance.

As the matrix gel (hydrogel) formed through hydrogen bonds can confine a pharmaceutical ingredient, even if a readily water-soluble pharmaceutical ingredient is employed, a resulting tablet would exhibit sustained-release performance.

A conventional method for tableting by compression may be employed as long as the pharmaceutical ingredient may be homogeneously embedded in the matrix PVA-based resin in the produced tablet.

For specifically exemplified tableting methods, a tableting by compression such as a direct powder compression (direct compression), a semi-dry granules compression, granules compression, or a tableting by wet molding process may be employed.

The direct powder compression is a method of directly mixing a pharmaceutical ingredient and other materials, and pressing the mixture in the absence of water. The dry granules compression is a method of granulating by dry process (or slugging) and then tableting by compression. The dry granules compression may achieve higher uniformity in the resultant tablet than the direct powder compression. Each of the semi-dry granule compression and the semi-direct compression is a method of preparing a granule containing materials other than a pharmaceutical ingredient by wet process and thereafter adding the pharmaceutical ingredient, followed by tableting. The use of granules prepared by wet process may enhance a cohesive strength in a resulting tablet, which can prevent powdering on a surface of the tablet. The wet granule compression is a method of preparing a granule containing a pharmaceutical ingredient and additives by wet process and then tableting the granules. The tableting by wet molding process is a method of molding or punching out the wet blend into a desirable shape, and then drying to remove a solvent such as water from the shaped product.

A mixture (composition for preparing granule) containing an additive in addition to the binder PVA-based resin and the pharmaceutical ingredient may be applied to the granulation process.

Examples of the granulation process include a wet granulation, dry granulation and spray-dry granulation. The wet granulation includes a granulation process of the mixture together with a binder solution, and a granulation process of wet lump obtained by kneading the mixture. The dry granulation is a granulation process comprising grinding a lump of compressed dry powder. The spray-dry granulation is a granulation process comprising preparing a slurry of powder with use of a large amount of water, spraying the slurry and drying to form granules.

The solvent used for a binder is appropriately selected according to the type of compound of the binder. In the case of a binder PVA-based resin, water can be used as a solvent.

As a granulator, a dry granulator, a basket-type extrusion granulator, a stirring granulator, a centrifugal tumbling granulator, a fluid bed granulator, spray drying granulator and the like may be used. The dry granulator is a machine for performing a dry granulation by applying a high pressure to a powder blend to create a lump, breaking down the lump to obtain powder having an appropriate particle size. The stirring granulator is a machine for granulation by mixing the powder ingredients with a stirring blade for several minutes, and adding a binder solution dropwise to perform granulation while stirring. The fluid bed granulator is a machine for granulation which comprises mixing powder ingredients fluidized with air to form a powder blend and spraying a binder solution with a nozzle in a convection or countercurrent flow to help the powder blend for agglomeration by spray droplets, and then drying to enlarge the size of the agglomerate. The spray drying granulator is a machine for performing granulation by spray drying.

In a typical wet granulation, ingredients are kneaded with a solvent and then granulated. Examples of this wet granulation include a granulation by milling, granulation by extrusion, granulation by stirring, granulation by tumbling process, and the like. For the wet granulation, an alcohol such as ethanol and isopropanol, or water may be use as the solvent.

Subsequently, thus prepared granules are pressed to form a tablet. The matrix PVA-based resin and the crystalline cellulose are preferably mixed with the granules in the tableting process. Adding a lubricant to the combination of the matrix PVA-based resin and the crystalline cellulose in the mixing process may improve compressibility, which is preferable.

A tableting by compression may be performed with use of a machine conventionally known in the pharmaceutical field, such as rotary tableting machine, a single punch tableting machine, or the like. For example, a tablet may be formed by a direct powder compression in which ingredients to be contained are evenly mixed and then compressed. A wet granule compression or a dry granule compression in which granules prepared from materials by wet granulation or dry granulation are compressed to form a tablet may be also applied. Among these tableting operations, the wet granule compression is preferred because of smoother fluidity and higher uniformity during the mixing process.

A tableting pressure, which is a compression force applied in compression molding, is preferably about 1 kN or more, more preferably about 5 kN or more, even more preferably about 10 kN or more, and may be 20 kN or more. A higher compression force may produce a tablet having a higher hardness. On the other hand, in order to avoid a defect such as tablet cracking, the upper limit of the compression force is preferably about 60 kN or less, more preferably about 50 kN or less, and even more preferably about 40 kN or less.

The pharmaceutical tablet may be ellipsoidal, cylindrical, spherical, doughnut-shaped, or any other shaped. Moreover, the tablet may be coated with a film, according to necessity.

A normal sustained-release tablet manufactured by above-mentioned processes has a hardness of 30 to 250 N, depending on a shape or size of the tablet, and may satisfy the properties required for a sustained-release tablet, especially a sustained-release tablet for oral administration. The sustained-release tablet with a diameter of 11 mm has a hardness of 30 to 200 N, preferably 50 to 200 N, more preferably 70 to 200 N.

Regarding the release behavior in accordance with a dissolution test based on DISSOLUTION Test 1 of USP41 "Metformin Hydrochloride Extended-Release Tablets", the released amount of pharmaceutical ingredient from the sustained-release tablet of the disclosure can fall in the following ranges. The dissolution test was conducted by dissolving 500 mg of the sustained-release tablet at 37°C in 1000 mL of phosphate buffer (pH 6.8), with use of a dissolution tester NTR-6400ACT (manufactured by Toyama Sangyo Co., Ltd.). The release rate of pharmaceutical ingredient from the tablet after 1 hour and 3 hours from the start of measurement was calculated on the basis of the dissolution rate after 10 hours as 100%. The dissolution rate reached plateau or almost steady state after 10 hours from the start of measurement, which can be assumed as 100% of the dissolution rate.

Release rate of pharmaceutical ingredient after 1 hour from the start of measurement: 20 to 55%, preferably 30 to 50%.
Release rate of pharmaceutical ingredient after 3 hours from the start of measurement: 45 to 85%, preferably 50 to 80%.
Release rate of pharmaceutical ingredient after 10 hours from the start of measurement: 90% or more, preferably 92% or more.

## EXAMPLES

The present invention will be further described below with reference to examples and comparative examples, but the present invention is not limited to the following examples.

[Measurement and evaluation method]

(1) Tablet hardness (N)

A hardness of a tablet manufactured by compression was measured using a PC-30 hardness tester (manufactured by OKADA SEIKO.CO., LTD.).

(2) Release rate

Dissolution Test 1 of USP41 "Metformin Hydrochloride Extended-Release Tablets" was conducted on a tablet containing metformin hydrochloride which is a readily water-soluble pharmaceutical ingredient.

Specifically, the dissolution test was conducted by dissolving 500 mg of tablets in 1000 mL of pH 6.8 phosphate buffer at 37°C and measuring the release rate (%) of the pharmaceutical ingredient with a dissolution tester NTR-6400ACT (manufactured by Toyama Sangyo Co., Ltd.) after 1 hour, 3 hours, and 10 hours from the start of the test.

[Materials used in Example]

(a) Pharmaceutical ingredient

Metformin hydrochloride was used. This pharmaceutical ingredient has high solubility and low membrane permeability, and is classified into class III in accordance with BCS classification.

(b) PVA-based resin for binder

An unmodified PVA having a saponification degree of 88 mol% and a viscosity of 3.4 mPa • s in 4% aqueous solution was used for a binder.

(c) PVA-based resin for matrix

Unmodified PVAs each having an average particle size ($\mu$m) and a viscosity in 4% aqueous solution which are indicated in Table 1, were used.

(d) Crystalline cellulose

Crystalline cellulose having an average particle size ($\mu$m) as indicated in Table 1 was used.

[Production of tablet Nos. 1 to 14]

1.1 parts by weight of the PVA-based resin for binder was dissolved in water to prepare PVA aqueous solution having a concentration of 20%. The PVA aqueous solution was added to 47.7 parts by weight of metformin hydrochloride (pharmaceutical ingredient), and then a granule of the pharmaceutical ingredient was produced by a granulator ("FM-VG-01" manufactured by Powrex Corporation).

The granule was mixed with a combination of the PVA-based resin for matrix and the crystalline cellulose in the presence of 1 part by weight of magnesium stearate. Table 1 shows an average particle size, tapped density, viscosity in 4% aqueous solution, and added amount of the matrix PVA-based resin used respectively, and also shows an average particle size, tapped density, viscosity in 4% aqueous solution, and added amount of the crystalline cellulose used respectively. The obtained mixture was compressed with a rotary tableting machine ("HT-EX12SS-U" manufactured by Hata Iron Works Co., Ltd.) at a compression force of 25 N to prepare a pharmaceutical tablet with 11 mm in diameter and 500 mg in weight.

The obtained tablets were measured with respect to tablet hardness and release rate according to the evaluation methods described above. The results are also shown in Table 1.

[Table 1]

| No | PVA for matrix | | | | crystalline cellulose | | | mixture | | release rate* [1] (%) | | | tablet hardness (N) |
|---|---|---|---|---|---|---|---|---|---|---|---|---|---|
| | average particle size Dc (μm) | tapped density (g/mL) | viscosity in 4% aqueous solution (mPa·s) | amount added (part) | average particle size Dp (μm) | Dc/Dp | amount added (part) | tapped density (g/mL) | content ratio PVA/Cel | 1h | 3h | 10h | |
| 1 | 46 | 0.621 | 49.5 | 46.3 | 90 | 2.0 | 5 | 0.613 | 9.26 | 48.6 | 78.9 30.3 | 95.8 16.9 | 75 |
| 2 | 46 | 0.621 | 49.5 | 46.3 | 170 | 3.7 | 5 | 0.61 | 9.26 | 42.9 | 71.2 28.3 | 90.4 19.2 | 86 |
| 3 | 46 | 0.621 | 49.5 | 46.3 | 20 | 0.43 | 5 | 0.618 | 9.26 | 45.4 | 76.3 30.9 | 92.8 16.5 | 136 |
| 4 | 83 | - | 49.5 | 46.3 | 90 | 1.08 | 5 | 0.622 | 9.26 | 54.2 | 82.9 28.7 | 100 17.1 | 34 |
| 5 | 78 | 0.676 | 49.5 | 46.3 | 90 | 1.15 | 5 | 0.628 | 9.26 | 50.7 | 81.7 31 | 95.3 13.6 | 39 |
| 6 | 97 | 0.683 | 49.5 | 46.3 | 90 | 0.93 | 5 | 0.632 | 9.26 | 44.2 | 71.1 26.9 | 92.3 21.2 | 30 |
| 7 | 111 | 0.691 | 49.5 | 46.3 | 90 | 0.81 | 5 | 0.629 | 9.26 | 50.2 | 81.1 30.9 | 93.2 12.1 | 18 |
| 8 | 62 | - | 43.5 | 46.3 | 90 | 1.45 | 5 | 0.656 | 9.26 | 45.5 | 80.1 34.6 | 89.2 9.1 | 19 |
| 9 | 55 | - | 5.5 | 46.3 | 90 | 1.63 | 5 | 0.675 | 9.26 | 76.3 | 97.3 21 | 97.7 0.4 | 31 |
| 10 | 122 | - | 30.2 | 46.3 | 90 | 0.73 | 5 | 0.627 | 9.26 | 51.9 | 79.6 27.7 | 89.1 9.5 | 21 |
| 11 | 46 | 0.621 | 49.5 | 34.2 | 90 | 2.0 | 17.1 | 0.560 | 2 | 50.9 | 89.8 38.9 | 99.4 9.6 | 149 |
| 12 | 46 | 0.621 | 49.5 | 25.65 | 90 | 2.0 | 25.65 | 0.524 | 1 | 47.6 | 83.4 35.8 | 94.4 11 | 158 |
| 13 | 46 | 0.621 | 49.5 | 39.46 | 90 | 2.0 | 11.84 | 0.576 | 3.33 | 37.2 | 67.1 29.9 | 93.8 26.7 | 155 |
| 14 | 46 | 0.621 | 49.5 | 42.75 | 90 | 2.0 | 8.55 | 0.589 | 5 | 34.9 | 61.5 | 93.8 | 123 |

(continued)

| No | PVA for matrix | | | | crystalline cellulose | | | mixture | | release rate* [1] (%) | | | tablet hardness (N) |
|---|---|---|---|---|---|---|---|---|---|---|---|---|---|
| | average particle size Dc ($\mu$m) | tapped density (g/mL) | viscosity in 4% aqueous solution (mPa·s) | amount added (part) | average particle size Dp ($\mu$m) | Dc/Dp | amount added (part) | tapped density (g/mL) | content ratio PVA/Cel | 1h | 3h | 10h | |
| | | | | | | | | | | | 26.6 | 32.3 | |

Regarding the release rate after 3 hours and 10 hours in Table 1, the upper row indicates the release rate (*1) at respective time, and the lower row indicates an increase amount (%) in release rate.

Nos. 1 to 6 are tablets produced with use of PVA-based resin having an average particle size of less than 100 μm and having a viscosity of 45 mPa • s or more measured in 4% aqueous solution. Tablets Nos. 1 to 6 exhibited that the increase amount in the release rate after 3 hours could be suppressed by about 30% as small as the release rate after 1 hour, but the release rate after 10 hours could reach up to 90% or more, preferably 95% or more. From these results, almost all pharmaceutical ingredients could be released with ensuring a sustained-release property.

Nos. 8 to 10 which were tablets produced using a matrix PVA-based resin with a low viscosity in 4% aqueous solution, had a tendency of lower hardness.

In addition, the tablet No. 9 having a notably low viscosity in 4% aqueous solution exhibited a high initial release rate, which failed to perform a sustained-release property. This result may be caused from insufficient network structure of the gel formed by the matrix PVA.

Each of tablets Nos. 1 to 7 was produced with use of the matrix PVA-based resin having the same average polymerization degree. However, if the ratio of the average particle size (Dc) of the crystalline cellulose and the average particle size (Dp) of the matrix PVA is apart from 1, for example, if Dc/Dp is either not less than 3 or not more than 0.5, the tablet hardness tended to be increased. This can be understood that the tablet produced by compression may have a reduced porosity. It is presumed that particles of one ingredient could intrude into a gap between particles of the other ingredient.

In this respect, Nos. 4 to 7 having a Dc/Dp close to 1, for example within the range of about 0.8 to 1.2, is difficult to achieve a compact state even in a tablet produced by compression. This may fail to achieve a sufficient tablet hardness in the production of the tablet by compression.

The tablet hardness seems to be influenced by the content ratio of the matrix PVA-based resin and the crystalline cellulose. From the comparison among Nos. 1 and 11 to 14, the closer the content ratio of the two (PVA : cellulose) approaches 1:1, the more the tablet hardness tended to be increased. In the case that the average particle size relationship (Dc/Dp) is 2, the closer the content of the matrix PVA-based resin is equal to the content of the crystalline cellulose, the broader the particle size distribution and the less the porosity in the tablet produced by compression. However, Nos. 11 and 12, in which the content ratio (PVA/Cel) was 2 or less, the matrix PVA-based resin could not remarkably contribute to a sustained-release performance. In these cases, the release rate within 3 hours was relatively high. The increased amount after 3 hours in release rate was more than 35%.

A sustained-release tablet was produced in the same manner as in No. 1 except that metformin hydrochloride was replaced with ascorbic acid which was classified into class I, and the release rate and tablet hardness were measured. The results are shown in Table 2. The release rate tended to be relatively high within 1 hour corresponding to an initial period of administration, but the increased amount in the release rate after 3 hours was about 30%, which was almost similar to No. 1.

[Table 2]

| No | PVA for matrix | | | | crystalline cellulose | | | mixture | | release rate (%) | | | tablet hardness (N) |
|---|---|---|---|---|---|---|---|---|---|---|---|---|---|
| | average particle size Dc (μm) | tapped density (g/mL) | viscosity in 4% aqueous solution (mPa·s) | amount added (part) | average particle size Dp (μm) | Dc/Dp | amount added (part) | tapped density (g/mL) | content ratio PVA/Cel | 1h | 3h | 10h | |
| 1 | 46 | 0.621 | 49.5 | 46.3 | 90 | 2.0 | 5 | 0.613 | 9.26 | 56.2 | 86.2 30.0 | 95.9 9.7 | 75 |

EP 4 393 512 B1

**INDUSTRIAL APPLICABILITY**

The pharmaceutical tablet of the present invention has an increased tablet hardness without impairing a sustained-release property of a pharmaceutical ingredient such as suppression of the release during the initial period of administration and achievement of a desired high release rate within 10 hours from the administration. Therefore, a composition for pharmaceutical tablet of the invention may ensure to provide a satisfactory sustained-release tablet even containing a readily soluble pharmaceutical ingredient classified into BCS classes I or III.

**Claims**

1. A composition for a pharmaceutical tablet comprising a polyvinyl alcohol-based resin and crystalline cellulose, wherein the polyvinyl alcohol-based resin has an average particle size of 100 $\mu$m or less and a viscosity greater than 44 mPa • s in 4% aqueous solution, the viscosity measured by the falling ball viscometer method according to JIS K6726.

2. The composition for a pharmaceutical tablet according to claim 1, wherein the crystalline cellulose has an average particle size of 250 $\mu$m or less and is either not greater than 0.5 times or not less than twice the size of the average particle size of the polyvinyl alcohol-based resin.

3. The composition for a pharmaceutical tablet according to claim 1 or 2, wherein the weight ratio of the polyvinyl alcohol-based resin and the crystalline cellulose, polyvinyl alcohol-based resin/crystalline cellulose, is in the range of 100/200 to 100/1.

4. The composition for a pharmaceutical tablet according to claim 1 or 2, wherein the weight ratio of the polyvinyl alcohol-based resin and the crystalline cellulose, polyvinyl alcohol-based resin/crystalline cellulose, is in the range of more than 2/1 to 50/1.

5. The composition for a pharmaceutical tablet according to claim 1 or 2, wherein the polyvinyl alcohol-based resin and the crystalline cellulose are blended so as to have a tapped density of 0.50 to 0.68 g/mL.

6. A composition for a pharmaceutical tablet comprising

   a polyvinyl alcohol having an average particle size of 100 $\mu$m or less and a viscosity greater than 44 mPa • s in 4% aqueous solution, the viscosity measured by the falling ball viscometer method according to JIS K6726; and
   a crystalline cellulose having an average particle size of 250 $\mu$m or less,
   wherein the average particle size of the crystalline cellulose is either not greater than 0.5 times or not less than twice the size of the average particle size of the polyvinyl alcohol-based resin, and
   wherein a ratio in weight of the polyvinyl alcohol-based resin and the crystalline cellulose, polyvinyl alcohol-based resin/crystalline cellulose, is in the range of more than 2/1 to 50/1.

7. The composition for a pharmaceutical tablet according to any one of claims 1 to 6, wherein the polyvinyl alcohol-based resin has a saponification degree of 70 mol% or more to 100 mol% or less.

8. A pharmaceutical tablet comprising a composition for a pharmaceutical tablet according to any one of claims 1 to 7 and a pharmaceutical ingredient classified into class I or III in accordance with BCS classification.

9. The pharmaceutical tablet according to claim 8, being a sustained-release tablet.

10. A method for producing a pharmaceutical tablet, the method comprising
    tableting a mixture containing a pharmaceutical ingredient, crystalline cellulose, and a polyvinyl alcohol-based resin having an average particle size of 100 $\mu$m or less and a viscosity of more than 44 mPa • s in 4% aqueous solution, the viscosity measured by the falling ball viscometer method according to JIS K6726.

11. The method for producing a pharmaceutical tablet according to claim 10, wherein the pharmaceutical ingredient is a granule granulated using a binder.

**Patentansprüche**

1. Eine Zusammensetzung für eine pharmazeutische Tablette, umfassend ein Harz auf Polyvinylalkohol-Basis und kristalline Cellulose,

wobei das Harz auf Polyvinylalkohol-Basis eine mittlere Teilchengröße von 100 µm oder weniger und eine Viskosität von mehr als 44 mPa · s in 4%iger wässriger Lösung aufweist, wobei die Viskosität gemäß JIS K6726 mit Hilfe des Kugelfallviskosimeterverfahrens gemessen wird.

2. Die Zusammensetzung für eine pharmazeutische Tablette nach Anspruch 1, wobei die kristalline Cellulose eine mittlere Teilchengröße von 250 µm oder weniger aufweist und entweder nicht größer als das 0,5-Fache oder nicht kleiner als das Doppelte der mittleren Teilchengröße des Harzes auf Polyvinylalkohol-Basis ist.

3. Die Zusammensetzung für eine pharmazeutische Tablette nach Anspruch 1 oder 2, wobei das Gewichtsverhältnis des Harzes auf Polyvinylalkohol-Basis und der kristallinen Cellulose, Harz auf Polyvinylalkohol-Basis/kristalline Cellulose, im Bereich von 100/200 bis 100/1 liegt.

4. Die Zusammensetzung für eine pharmazeutische Tablette nach Anspruch 1 oder 2, wobei das Gewichtsverhältnis des Harzes auf Polyvinylalkohol-Basis und der kristallinen Cellulose, Harz auf Polyvinylalkohol-Basis/kristalline Cellulose, im Bereich von mehr als 2/1 bis 50/1 liegt.

5. Die Zusammensetzung für eine pharmazeutische Tablette nach Anspruch 1 oder 2, wobei das Harz auf Polyvinylalkohol-Basis und die kristalline Cellulose derart gemischt sind, dass sie eine Klopfdichte von 0,50 bis 0,68 g/ml aufweisen.

6. Eine Zusammensetzung für eine pharmazeutische Tablette, umfassend

ein Polyvinylalkohol mit einer mittleren Teilchengröße von 100 µm oder weniger und einer Viskosität von mehr als 44 mPa · s in 4%iger wässriger Lösung, wobei die Viskosität mit Hilfe des Kugelfallviskosimeterverfahrens gemäß JIS K6726 gemessen wird; und
eine kristalline Cellulose mit einer mittleren Teilchengröße von 250 µm oder weniger,
wobei die mittlere Teilchengröße der kristallinen Cellulose entweder nicht größer als das 0,5-Fache oder nicht kleiner als das Doppelte der mittleren Teilchengröße des Harzes auf Polyvinylalkohol-Basis ist, und
wobei ein Gewichtsverhältnis des Harzes auf Polyvinylalkohol-Basis und der kristallinen Cellulose, Harz auf Polyvinylalkohol-Basis/kristalline Cellulose, im Bereich von mehr als 2/1 bis 50/1 liegt.

7. Die Zusammensetzung für eine pharmazeutische Tablette nach einem der Ansprüche 1 bis 6, wobei das Harz auf Polyvinylalkohol-Basis einen Verseifungsgrad von 70 Mol-% oder mehr bis 100 Mol-% oder weniger aufweist.

8. Eine pharmazeutische Tablette, umfassend eine Zusammensetzung für eine pharmazeutische Tablette nach einem der Ansprüche 1 bis 7 und einen pharmazeutischen Bestandteil, der gemäß der BCS-Klassifizierung in Klasse I oder III eingestuft ist.

9. Die pharmazeutische Tablette nach Anspruch 8, bei welcher es sich um eine Tablette mit verzögerter Freisetzung handelt.

10. Ein Verfahren zur Herstellung einer pharmazeutischen Tablette, wobei das Verfahren umfasst:
Tablettieren eines Gemischs, das einen pharmazeutischen Bestandteil, kristalline Cellulose und ein Harz auf Polyvinylalkohol-Basis mit einer mittleren Teilchengröße von 100 µm oder weniger und einer Viskosität von mehr als 44 mPa · s in 4%iger wässriger Lösung enthält, wobei die Viskosität mit Hilfe des Kugelfallviskosimeterverfahrens gemäß JIS K6726 gemessen wird.

11. Das Verfahren zur Herstellung einer pharmazeutischen Tablette nach Anspruch 10, wobei der pharmazeutische Bestandteil ein Granulat ist, das unter Verwendung eines Bindemittels granuliert wird.

**Revendications**

1. Composition pour un comprimé pharmaceutique comprenant une résine à base d'alcool polyvinylique et de cellulose cristalline,
dans laquelle la résine à base d'alcool polyvinylique a une taille moyenne de particule de 100 μm ou moins et une viscosité supérieure à 44 mPa · s dans une solution aqueuse à 4 %, la viscosité étant mesurée par la méthode du viscosimètre à chute de bille selon la norme JIS K6726.

2. Composition pour un comprimé pharmaceutique selon la revendication 1, dans laquelle la cellulose cristalline a une taille moyenne de particule de 250 μm ou moins et n'est soit pas supérieure à 0,5 fois soit pas inférieure à deux fois la taille de la taille moyenne de particule de la résine à base d'alcool polyvinylique.

3. Composition pour un comprimé pharmaceutique selon la revendication 1 ou 2, dans laquelle le rapport en poids de la résine à base d'alcool polyvinylique et de la cellulose cristalline, résine à base d'alcool polyvinylique/cellulose cristalline, est situé dans la plage de 100/200 à 100/1.

4. Composition pour un comprimé pharmaceutique selon la revendication 1 ou 2, dans laquelle le rapport en poids de la résine à base d'alcool polyvinylique et de la cellulose cristalline, résine à base d'alcool polyvinylique/cellulose cristalline, est situé dans la plage de plus de 2/1 à 50/1.

5. Composition pour un comprimé pharmaceutique selon la revendication 1 ou 2, dans laquelle la résine à base d'alcool polyvinylique et la cellulose cristalline sont mélangées de manière à avoir une densité tassée de 0,50 à 0,68 g/ml.

6. Composition pour un comprimé pharmaceutique comprenant

   un alcool polyvinylique ayant une taille moyenne de particule de 100 μm ou moins et une viscosité supérieure à 44 mPa · s dans une solution aqueuse à 4 %, la viscosité étant mesurée par la méthode du viscosimètre à chute de bille selon la norme JIS K6726 ; et
   une cellulose cristalline ayant une taille moyenne de particule de 250 μm ou moins,
   dans laquelle la taille moyenne de particule de la cellulose cristalline n'est soit pas supérieure à 0,5 fois soit pas inférieure à deux fois la taille de la taille moyenne de particule de la résine à base d'alcool polyvinylique, et
   dans laquelle un rapport en poids de la résine à base d'alcool polyvinylique et de la cellulose cristalline, résine à base d'alcool polyvinylique/cellulose cristalline, est situé dans la plage de plus de 2/1 à 50/1.

7. Composition pour un comprimé pharmaceutique selon l'une quelconque des revendications 1 à 6, dans laquelle la résine à base d'alcool polyvinylique a un degré de saponification de 70 % en moles ou plus à 100 % en moles ou moins.

8. Comprimé pharmaceutique comprenant une composition pour un comprimé pharmaceutique selon l'une quelconque des revendications 1 à 7 et un ingrédient pharmaceutique classé en classe I ou III conformément à la classification BCS.

9. Comprimé pharmaceutique selon la revendication 8, qui est un comprimé à libération prolongée.

10. Méthode de production d'un comprimé pharmaceutique, la méthode comprenant
    la compression d'un mélange contenant un ingrédient pharmaceutique, de la cellulose cristalline et une résine à base d'alcool polyvinylique ayant une taille moyenne de particule de 100 μm ou moins et une viscosité supérieure à 44 mPa · s dans une solution aqueuse à 4 %, la viscosité étant mesurée par la méthode du viscosimètre à chute de bille selon la norme JIS K6726.

11. Méthode de production d'un comprimé pharmaceutique selon la revendication 10, dans laquelle l'ingrédient pharmaceutique est un granule granulé en utilisant un liant.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016013675 A

- JP 2018530537 A